# EUROPEAN PATENT APPLICATION

(11) **EP 2 918 281 A1**
(43) Date of publication of application: **16.09.2015**
(21) Application number: 14159534.8
(22) Date of filing: 13.03.2014
(51) Int. Cl.: A61K 36/81, A61K 38/56, A61P 19/10

(54) **Novel uses of potato tuber proteinaceous inhibitors**

(71) Applicant: Max-Planck-Gesellschaft zur Förderung der Wissenschaften e.V., 80539 München (DE)
(72) Inventor: Gebhardt, Christiane, 53902 Bad Münstereifel (DE); Fischer, Matthias, 44289 Dortmund (DE)
(74) Representative: Arth, Hans-Lothar

(57) **Abstract**

The present invention relates to inventive uses of potato tuber derived proteinaceous protease inhibitor, especially for use as an inhibitor of cathepsin K, 5-lipoxygenase, HIV-Protease, and human β-secretase. These enzymes are involved in severe human diseases. Therefore, the potato tuber derived proteinaceous protease inhibitor described herein can be used for the treatment of osteoporosis, allergies, asthma bronchiale, HIV-infection, and Alzheimer's disease.

## Description

The present invention relates to inventive uses of potato tuber derived proteinaceous protease inhibitor, especially for use as an inhibitor of cathepsin K, 5-lipoxygenase, HIV-Protease, and human β-secretase. These enzymes are involved in severe human diseases. Therefore, the potato tuber derived proteinaceous protease inhibitor described herein can be used for drug development to treat osteoporosis, allergies, asthma, HIV-infection, and Alzheimer's disease.

### Background of the invention

Protease inhibitors (PIs) are ubiquitous, small proteins which are particularly abundant in plant reproductive and storage organs such as seeds or tubers. They account for 1% to 10% of the total protein in storage tissues. In higher plants, several PI families have been characterized regarding their molecular and biochemical function. The structural basis of the interaction between proteases and their inhibitors is under continuous investigation. PIs are involved in many physiological processes via control of protease activity. The best characterized example is their role in wound induced defense responses of plants against herbivores and pathogens. They are also considered as storage proteins.

Potato tuber protein consists mainly of patatin, the major storage protein, and numerous low molecular weight PIs which can be classified in at least ten different families. Most abundant are inhibitors of serine proteases from families known as Kunitz-type inhibitors (KTI), potato protease inhibitors I and II (PIN I, PIN II) and Bowman-Birk inhibitors (BBIs). KTIs are one of the best characterized inhibitor families. They are abundant in potato tubers and represent a highly diverse group of proteins. Most KTIs are encoded at a complex locus on potato chromosome III, which is linked to a quantitative trait locus (QTL) for resistance to the oomycete *Phytophtora infestans.* Most KTIs consist of a single polypeptide chain of approximately 24 kDa with two disulfide bridges and a single reactive site. Depending on the studied cultivar, potato KTIs were classified in three to six structural subgroups (A, B, C, D, K and M). Previous studies revealed that KTIs have distinct target specificities and some have dual or broad specificity. Inhibitors of family KTI-A reduce the activity of serine or aspartic proteases such as trypsin or cathepsin D. Members of subgroup KTI-B inhibit trypsin, chymotrypsin or elastase. Similar to KTIs, the PIN I and PIN II families display high structural and functional diversity, particularly in the Solanaceae. They are organized as gene clusters mainly on potato chromosome IX and III, respectively. A number of plant PINs have been characterized at the biochemical and molecular level. PIN I protein was first isolated from potato tubers. More recent studies demonstrated PIN I expression in leaves, stems, flowers and tuber sprouts, which is regulated by both environmental and developmental signals. Proteins homologous to potato PIN I are found in several plant species such as barley or maize, while PIN II's seem to be restricted to the Solanaceae. PIN proteins are suggested to function in the plant interaction with herbivores and microbes. Digestive enzymes in the guts of herbivores were inhibited by plant PINs, restricting the absorption of essential amino acids and consequently interfering with herbivore growth and development. In vitro assays confirmed inhibitory effects of plant PINs on the digestive serine proteases trypsin, chymotrypsin or subtilisin.

The detrimental effect observed on herbivores and pests led to the development of inhibitor-transgenic plants. However, due to the adaptation of herbivores by maintaining diverse digestive enzymes and over-expressing inhibitor insensitive enzymes, and last but not least due to the rejection of transgenic crops by the public, transgenic approaches have not been widely adopted in commercial food crops. Beyond plant biotechnology, plant PIs became attractive targets in pharmacology and drug development. Inhibitors of KTI and BBI families, purified from different leguminous seeds, were shown to block the activity of several proteases and enzymes involved in human diseases (reviewed in Oliva MLV, Sampaio MU: Action of plant proteinase inhibitors on enzymes of physiopathological importance. Anais Da Academia Brasileira De Ciencias 2009, 81 (3):615-621). Plant KTIs inhibit proteins acting in the blood clotting cascade or in fibrinolysis such as factor XIIa, factor Xa, thrombin, plasmin, plasma kallikrein or tissue plasminogen activator. Elastase and cathepsin G involved in inflammatory processes in humans were shown to be inhibited by KTIs isolated from Bauhinia seeds. Several studies revealed anti-tumor activity of BBI and KTI inhibitors. In summary, KTI effects were investigated in cancer, inflammation, thrombosis, and antifungal activity.

It is the objective of the present invention to provide novel pharmaceutical targets of potato tuber proteinaceous inhibitors and therefore novel and inventive medical uses for this class of proteins.

The objective of the present invention is solved by the teaching of the independent claims. Further advantageous features, aspects and details of the invention are evident from the dependent claims, the description, the figures, and the examples of the present application.

### Description of the invention

The inventors tap at the tremendous structural diversity of proteinaceous inhibitors found in tubers of different potato genotypes and their potential inhibitory functions. Novel variants of proteinaceous inhibitors were isolated as full length cDNAs from tubers of ten different potato varieties. A subset was expressed as recombinant fusion proteins in the yeast *Pichia pastoris* and examined for inhibitory properties on different enzymes involved in human diseases including Alzheimer disease, AIDS, asthma, allergic reactions and osteoporosis. Proteinaceous inhibitors of the Kunitz-type inhibitor (KTI) and potato protease inhibitor I and II (PIN I and II) families were found to inhibit cathepsin K, 5-lipoxygenase, HIV-protease, human β-secretase, and pig pancreas elastase.

Thus the present invention relates to potato tuber derived proteinaceous protease inhibitor for use in medicine, especially as an inhibitor of cathepsin K, 5-lipoxygenase, HIV-protease, and/or human β-secretase. These protease inhibitors are preferably selected from the group of protease inhibitors consisting of: KPI-B inhibitors, KPI-C inhibitors, PIN I inhibitors and PIN II inhibitors. The present invention refers also to the use, preferably *in vitro,* of potato tuber derived proteinaceous protease inhibitor as an inhibitor of cathepsin K, 5-lipoxygenase, HIV-protease and/or human β-secretase. This use is suitable in the treatment of different disorders but may also be advisable for scientific reasons and useful as starting material for the development or design of novel active agents. Enzyme inhibitors are, in general, molecules which bind to enzymes and decrease their activity. The binding of an inhibitor can stop a substrate from entering the enzyme's active site, compete with the substrate for the binding site, or hinder the enzyme from catalyzing its reaction. Inhibitor binding can be reversible or irreversible. Proteinaceous protease inhibitors are proteins or polypeptides acting as regulators of endogenous proteolytic activity. Thereby proteinaceous protease inhibitors are peptidic molecules able to inhibit the function of proteases.

Cathepsin K is a lysosomal cysteine proteinase being important in osteoclast function and involved in bone remodeling and resorption. Cathepsin K represents a promising target for treating diseases characterized by excessive bone resorption, such as osteoporosis. The cathepsin K inhibitor odanacatib which is so far not approved showed in the phase III clinical trial high potential in the treatment of osteoporosis.

The 5-lipoxygenase (LOX, Arachidonate 5-lipoxygenase) activity converts arachidonic acid in 5-hydroperoxyeicosatetraenoic acid (5-HPETE), an intermediate in the biosynthesis of leukotrienes and other eicosanoids. Leukotrienes are a family of eicosanoid inflammatory mediators produced in leukocytes. They trigger contractions in the smooth muscles being present in the bronchioles; their overproduction is a major cause of inflammation in asthma bronchiale and allergic rhinitis. Leukotrienes antagonists as well as glucocorticoids (inhibiting unspecific formation of arachidonic acid derivatives) are used in treatment of asthma bronchiale; more recently a potential role also in atherosclerosis has raised considerable interest.

HIV-protease is a retroviral aspartyl protease essential for the life-cycle of human immunodeficiency virus (HIV). The precursor gag polyprotein of HIV is processed by HIV protease during maturation to different structural proteins of the capsid core particle. Without HIV protease activity, HI virions are not infectious. Different HIV-protease inhibitors are used as an antiretroviral drug in HIV therapy (such as saquinavir and indinavir).

β-secretase (also called beta-site amyloid beta A4 precursor protein-cleaving enzyme 1, BACE1) is an aspartic-acid protease involved in the formation of myelin sheaths in peripheral nerve cells. Deposition of amyloid beta peptide aggregates in the brain is an early and critical characteristic of Alzheimer's disease which depends on two sequential proteolytic cleavages of the amyloid precursor protein (APP) by β-secretase and γ-secretase. In a first step, extracellular cleavage by human β-secretase results in a soluble extracellular fragment and a cell membrane-bound fragment. γ-secretase cleaves subsequently within the membrane-spanning domain of the amyloid precursor protein (APP). Increased levels of β-secretase have been shown in the late-onset sporadic Alzheimer's disease. Therefore it is assumed that drugs blocking or inhibiting β-secretase would prevent formation of beta-amyloid and may help to slow down or stop Alzheimer's disease.

Therefore, potato tuber derived proteinaceous protease inhibitors, and in particular KPI-B inhibitors, KPI-C inhibitors, PIN I inhibitors and PIN II inhibitors are suitable for use in the treatment of osteoporosis, allergies, asthma bronchiale, HIV-infection, and Alzheimer's disease. In other words the present invention refers to the use of potato tuber derived proteinaceous protease inhibitors for the manufacture of a medicament for use in the treatment of osteoporosis, allergies, asthma bronchiale, HIV-infection, and Alzheimer's disease. One aspect of the present invention refers to a method of treating osteoporosis, allergies, asthma bronchiale, HIV-infection, or Alzheimer's disease comprising: administering to a subject who would benefit from such a treatment a therapeutically effective amount of a composition comprising a potato tuber derived proteinaceous protease inhibitor as described herein and optionally one or more pharmaceutically acceptable carriers.

The inhibitors might also be used as structural models for the chemical synthesis of small non-proteinaceous inhibitor molecules which are suitable for use in the treatment of osteoporosis, allergies, asthma bronchiale, HIV-infection, and Alzheimer's disease. Thus, one embodiment of the present invention refers to the use of the potato tuber derived proteinaceous protease inhibitors described herein for the identification, optimization and/or design of inhibitors of cathepsin K, 5-lipoxygenase, HIV-protease, and/or human β-secretase. Knowing the amino acid sequence of suitable inhibitors allows generating a three-dimensional model of the potato tuber derived proteinaceous protease inhibitor. A three-dimensional structure of a protein may be predicted using established computer software, too. One example is the protein structure homology-modelling server SWISS-MODEL. Such a model provides the possibility to design suitable inhibitors, identify them from a compound library or optimize a known suitable inhibitor by increasing the inhibitory potential. Design, identification and optimization of suitable inhibitors can be performed with standard computer based methods and software programs well known in the art.

A variety of commercially available software programs are available for conducting the analysis and comparison of data in the computer-based system. One skilled in the art will readily recognize which of the available algorithms or implementing software packages for conducting computer analyses can be utilized or adapted for use in the computer-based system.

Thus the present invention is also directed to a method for designing a compound that interacts with or inhibits cathepsin K, 5-lipoxygenase, HIV-protease, and/or human β-secretase, comprising the steps of
(a) generating a three-dimensional model of potato tuber derived proteinaceous protease inhibitor according to anyone of SEQ ID No. 1 - SEQ ID No. 14; and
(b) employing said three-dimensional model to design a compound that should have the ability to interact with or inhibit cathepsin K, 5-lipoxygenase, HIV-protease, and/or human β-secretase.

The term "compound" as used herein preferably refers to a small chemical molecule and in particular to a so called peptidomimetic. A peptidomimetic is a small protein-like chain designed to mimic a peptide. Thereby, the altered chemical structure is designed in order to improve stability or biological activity. The compounds obtained in accordance with step (b) above, therefore, contain preferably a peptidic or peptidomimetic backbone. These compounds should be suitable for use in the treatment of osteoporosis, allergies, asthma bronchiale, HIV-infection, and/or Alzheimer's disease.

Osteoporosis is a progressive bone disease characterized by a decrease in bone mass and density which often leads to an increased risk of fracture. An allergy is a hypersensitivity disorder of the immune system. Thereby a patient's immune system reacts to normally harmless substances as an allergen. Allergy is also called immediate (type I) hypersensitivity. Asthma or asthma bronchiale refers to a chronic inflammatory disease of the airways characterized by variable and recurring symptoms, such as reversible airflow obstruction and bronchospasm. The strongest risk factor for developing asthma is a history of atopic diseases, such as allergies. The term HIV-infection refers to an infection with the human immunodeficiency virus and all diseases caused by that infection including acquired immunodeficiency syndrome (AIDS). Alzheimer's disease (AD) is a common form of dementia among older people. The pathogenic mechanism and progression of Alzheimer's disease are not completely understood. Research indicates that the disease is associated with abnormal deposits of proteins form amyloid plaques and tau tangles. Current treatments only reduce the symptoms of the disease.

One preferred family of potato tuber derived proteinaceous protease inhibitors suitable for the use of the present invention is Kunitz-type inhibitor group C proteins (herein called KPI-C inhibitors or KPI-C proteins). One preferred embodiment of the present invention is a KPI-C protein for the use as an inhibitor of cathepsin K or 5-lipoxygenase. These KPI-C inhibitors are especially suitable for the use in the treatment of osteoporosis, allergies or asthma bronchiale. Particularly preferred is the use in the treatment of allergic asthma bronchiale. In other words the present invention refers to the use of KPI-C inhibitors for the manufacture of a medicament for use in the treatment of osteoporosis, allergies or asthma bronchiale.

According to the present invention potato tuber derived proteinaceous protease inhibitors according to SEQ ID No. 01, SEQ ID No. 02 and SEQ ID No. 03 are preferred KPI-C inhibitors. One embodiment of the present invention refers to the KPI-C inhibitor according to SEQ ID No. 02 and its use as an inhibitor of cathepsin K or 5-lipoxygenase. The protein according to SEQ ID No. 02 is in particular suitable for the treatment of osteoporosis, allergies and asthma bronchiale.

One preferred family of potato tuber derived proteinaceous protease inhibitors suitable for the use of the present invention is the family of potato protease inhibitors I (herein called PIN I inhibitor or PIN I protein). One preferred embodiment of the present invention is a PIN I protein for the use as an inhibitor of HIV-protease and cathepsin K. These PIN I inhibitors are especially suitable for the use in the treatment of HIV-infection and osteoporosis. In other words the present invention refers to the use of PIN I inhibitors for the manufacture of a medicament for use in the treatment of HIV-infection and osteoporosis.

According to the present invention potato tuber derived proteinaceous protease inhibitors according to SEQ ID No. 04 is a preferred PIN I inhibitor. The PIN I inhibitor according to SEQ ID No. 04 and its use as an inhibitor of cathepsin K and HIV-protease are each one embodiment of the present invention. The protein according to SEQ ID No. 04 is at least suitable for the treatment of HIV-infection and osteoporosis.

Another preferred family of potato tuber derived proteinaceous protease inhibitors suitable for the use of the present invention is the protein family of Kunitz-type inhibitor group B (herein called KPI-B inhibitors or KPI-B proteins). One preferred embodiment of the present invention is a KPI-B protein for the use as an inhibitor of human β-secretase (also called BACE I). These KPI-B inhibitors are especially suitable for the use in the treatment of Alzheimer's disease. Therefore, the present invention refers to the use of KPI-B inhibitors for the manufacture of a medicament for use in the treatment of Alzheimer's disease.

According to the present invention potato tuber derived proteinaceous protease inhibitors according to a sequence from SEQ ID No. 07 up to SEQ ID No. 13 are preferred KPI-B inhibitors. Preferred embodiments of the present invention are the KPI-B inhibitors according to SEQ ID No. 07, SEQ ID No. 08, SEQ ID No. 09 and SEQ ID No. 11 and their use as an inhibitor of human β-secretase.

The potato tuber derived proteinaceous protease inhibitors of the invention can be extracted from potato tuber. Alternatively, they may also be produced using recombinant DNA and commonly known techniques. Thereby, they may be produced and extracted from microbial cells, such as recombinant E. coli or yeast cultures, mammalian cell lines or plant cell cultures The critical feature is that the preparation allows for the desired function of the polypeptides, even in the presence of considerable amounts of other components. Thus, the invention encompasses various degrees of purity. When a polypeptide is recombinantly produced, it should be substantially free of culture medium, i.e., culture medium represents less than about 20%, less than about 10%, or less than about 5% of the volume of the polypeptide preparation used.

For use in medicine or as a medicament, the potato tuber derived proteinaceous protease inhibitors according to the present invention may be formulated as a pharmaceutical composition for the use in the treatment of osteoporosis, allergies, asthma bronchiale, HIV-infection, or Alzheimer's disease. Thus, another aspect of the present invention is directed to pharmaceutical compositions comprising at least one potato tuber derived proteinaceous protease inhibitors as described herein as active ingredient, together with at least one pharmaceutically acceptable carrier, excipient and/or diluent. The pharmaceutical compositions can be prepared in a conventional solid or liquid carrier or diluent at suitable dosage level in a known way.

Furthermore, the present invention also includes pharmaceutical composition or preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one proteinaceous protease inhibitor as described herein and/or a pharmaceutical acceptable salt thereof as active ingredient. In particular preferred is a composition adapted for injection.

The pharmaceutical compositions according to the present invention containing at least one potato tuber derived proteinaceous protease inhibitor as active ingredient will typically be administered together with suitable carrier materials selected with respect to the intended form of administration and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the potato tuber derived proteinaceous protease inhibitors described herein.

Suitable binders include starch, gelatin, natural carbohydrates, corn sweeteners, natural and synthetic gums such as acacia, sodium alginate, carboxymethylcellulose, polyethylene glycol and waxes. Among suitable lubricants, there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate.

Liquid form preparations include solutions, suspensions, and emulsions. As an example, there may be mentioned water or water/propylene glycol solutions for parenteral injections. Liquid form composition or preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

For liquid compositions, especially in the form of solutions for parenteral injections, the active drug component may be combined with additives for buffering, for preservation, for adjusting pH or osmolarity, and/or for providing stability.

Pharmaceutical compositions for injection, in particular for intravenous administration, typically are solutions in sterile isotonic aqueous buffer. Where necessary, the composition may also include a solubilizing agent and a local anesthetic to ease pain at the site of the injection.

The buffering agent to be used can be a sodium phosphate buffer, a citrate buffer, an acetate buffer or buffer based on Tris (tris(hydroxymethyl)aminomethane), such as TAE (Tris-acetate-EDTA) buffer. The buffer is preferably used in order to maintain a pH value in the range of about 5.0 to about 7.2, preferably from 5.9 to 6.5. The pH value may be adjusted with a corresponding base. The amount of the buffering agent in the pharmaceutical formulation may range from about 5 mM to about 50 mM. For adjusting pH of the pharmaceutical compositions acids or base may used, such as hydrochloric acid and sodium hydroxide.

Different sugars such as saccharose or trehalose may be added as cryoprotecting agents or for adjusting osmolarity of the solution. Polyalcohols, for example mannitol and sorbitol, may be used as stabilizer or for adjusting osmolarity.

Furthermore liquid form compositions comprise preferably also a non-ionic surfactant, preferably polysorbate. Polysorbates are the polycondensation products of sorbitane esters and polyethylene glycol. The polysorbates are commercially available, for example under tradenames Tween^{®} 20 or Tween^{®} 80, respectively.

Furthermore the composition may contain amino acids, such as glycine, arginine, leucine or proline, as stabilizer. Further stabilizer of the solution may be urea or human serum albumin. In addition salts, such as NaCl, may be added in low concentration for stabilization of liquid form preparations.

Benzyl alcohol may be used as a bacteriostatic preservative at low concentration in the composition and especially in liquid form preparations of the present invention. In addition antioxidants, such as methionine, may be used.

Also included are solid form preparations, which are intended to be converted, shortly before use, to liquid form compositions or preparations for either oral or parenteral administration. Such solid form preparations contain the potato tuber derived proteinaceous protease inhibitor for example as lyophilisate. Where the composition is to be administered by infusion, it can be dispensed with an infusion bottle containing sterile pharmaceutical grade water, saline or dextrose/water. Where the composition is administered by injection, an ampoule of sterile water for injection or saline can be provided so that the ingredients may be mixed prior to administration.

The 5-LOX pathway is not only relevant for inflammatory processes in humans but also for plant defense. Plant lipoxygenases (LOX) are key enzymes in the biosynthesis of oxilipins, which play important roles in plant defense, pest resistance and development. The substrates of LOX are polyunsaturated fatty acids. Trypsin and elastase are digestive proteases of insects that were previously shown to be targeted in the context of plant defense against herbivores. Two representatives from the potato proteinase inhibitor family II (PIN II) inhibited trypsin and in addition elastase from pig pancreas (PPE), alternative names are chymotrypsin-like elastase family member 1 and elastase-1. Therefore potato tuber derived proteinaceous protease inhibitor and in particular PIN II proteins function in planta presumably in defense reactions in response to the attack of herbivores and other pests. Thus, one aspect of the present invention is directed to potato tuber derived proteinaceous protease inhibitor and in particular PIN II proteins for use as pesticide, especially as insecticides.

Furthermore inhibitors of trypsin and elastase-1, in human, are suitable as anti-aging and skin-whitening ingredients in cosmetics. Thus, one preferred embodiment of the present invention refers to the use of potato tuber derived proteinaceous protease inhibitors, in particular PIN I inhibitor, PIN II inhibitor, KPI-B inhibitor or KPI-C inhibitor and preferably PIN II proteins and preferably of PIN II inhibitor, as ingredient, especially as anti-aging and skin-whitening compounds, for cosmetic formulations.

In addition, the present invention is related to cosmetic and dermatological compositions containing potato tuber derived proteinaceous protease inhibitors, and in particular PIN I inhibitor, PIN II inhibitor, KPI-B inhibitor or KPI-C inhibitor and preferably PIN II proteins, for anti-aging and skin-whitening treatments.

Under cosmetic and/or dermatological composition are particularly understood skin crèmes, skin lotions, emulsions, gels, suspensions, ointments, sticks, oils as well as balsams and all other formulations suitable for skin applications. These formulations can further contain any common carrier, excipient and, if necessary, further active agents. Preferred excipients are selected from the group comprising or consisting of preservatives, antioxidants, stabilizers, solubilizers, vitamins and fragrances.

### Description of the figure:

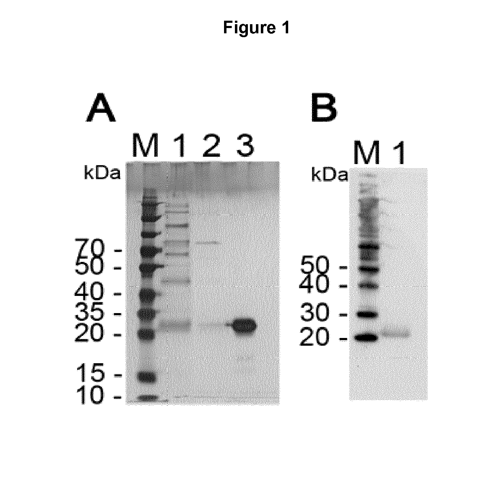

**Figure 1****:** Purification of the fusion protein containing inhibitor 01 from *P. pastoris* culture media by affinity chromatrography. (A) Coomassie stained SDS-PAGE. Lane *1, P. pastoris* culture media supernatant after 72h induction of inhibitor 01 expression by methanol. Lane 2, proteins precipitated with 40% ammonium sulfate from P. *pastoris* culture media. Lane 3, purified and concentrated inhibitor 01 protein. (B) Western blot analysis. Lane 1, purified and concentrated inhibitor 01 protein following incubation with anti-myc-tag antibody. M, molecular weight standards

### EXAMPLES

### Example 1: Preparation of cDNA libraries from different potato tuber varieties

Field grown tubers from nine potato varieties (Lady Claire, Omega, Eurobeta, Verdi, Elfe, Marabel, Allians, Solara, Melba) and one breeding clone (Breeding clone 18) were provided by EUROPLANT Pflanzenzucht GmbH (Lüneburg, Germany). Tubers were peeled, washed and snap frozen in liquid nitrogen. Tuber tissue was homogenized by Mixer Mill MM 200 (Retsch, Haan, Germany) and stored at -80°C until use.

Total RNA was isolated from 5g powdered, frozen tuber tissue from 10 potato genotypes using the PureLink® Plant-RNA reagent (Invitrogen™, Carlsbad, USA) according to the manufacturer's guidelines. Poly A⁺ RNA was prepared from total RNA using the Poly(A)PuriSt™ MAG purification kit (Ambion™, Austin, USA) following the supplier's protocol. Ten cDNA libraries were constructed from Poly A⁺ RNA of the 10 genotypes. cDNA synthesis and transformation of Electromax™ DH10B™ T1 Phage resistant *E. coli* cells were performed using to the CloneMiner™ II cDNA Library construction kit (Invitrogen™, Carlsbad, USA) and the supplier's protocol. 960 cDNA clones per library were randomly selected (9600 clones total) and stored in 96-well microtiter plates.

### Example 2: cDNA sequence analysis

Plasmid insertions were sequenced from the 5' end using a primer (5'GTAAAACGACGGCCAGT3') matching to the M13-forward (-20) priming site of the pDONR™222 vector which was used for cDNA library construction. Custom DNA Sanger-sequencing was performed at the Max-Planck Genome Center (Cologne, Germany, (http://mpgc.mpipz.mpg.de/home/)). Inhibitor sequences were identified by BLAST comparisons to the NCBI nucleotide collection (NR/NT). cDNA clones coding for various inhibitors of proteases and other enzymes were selected and sequenced from the 3' end using a primer (5'AACAGCTATGACCATG3') specific for the M13-reverse priming site, in order to identify full length cDNA clones. Recombinant *E. coli* clones harboring full length inhibitor sequences were propagated in LB-media (1% Tryptone, 0.5% yeast extract, 1% NaCl, pH 7) containing 50µg/ml kanamycin. Plasmid DNA was isolated using the Plasmid Mini Kit (QIAGEN®, Hilden, Germany) according to the manufacturer's guidelines and stored at -20°C.

### Example 3: Expression and purification of recombinant protein inhibitors

Full length cDNAs encoding protease inhibitors were cloned into the multiple cloning site (MCS) of the expression vector pPICZαA (Invitrogen™, Carlsbad, USA) without their putative signal sequences. Putative signal sequences were identified using SignalP 4.0 software (http://www.cbs.dtu.dk/services/SignalP/). Translation termination sequences were omitted in fusing inhibitor sequences with the c-myc epitope and His-tag of the pPICZαA vector. Expression of fusion proteins was controlled by the methanol-inducible AOX1 promoter. The native α-factor secretion signal sequence from *Saccharomyces cerevisiae* allowed secretion of the fusion protein to the media. For expression cloning, inhibitor sequences were amplified by PCR using 1 ng plasmid DNA as template and the primers specified in the sequence listing. PCR conditions were: 95°C for 5min, followed by 20 cycles of 95°C for 1 min, 60° for 1 min and 72°C for 1.5min with a final extension step at 72°C for 5min. Cloning of PCR products into the pPICZαA vector and transformation of *Pichia pastoris* cells (strain GS115) was accomplished with the EasySelect™ Pichia Expression Kit (Invitrogen™, Carlsbad, USA) according to the suppliers protocol. Nucleotide sequences were verified by direct sequencing.

Recombinant *P. pastoris* strains expressing a specific inhibitor were pre-cultured by inoculating 100ml BMGY medium (1% w/w yeast extract, 2% w/w peptone, 100mM potassium phosphate pH 6, 1.34% w/w yeast nitrogen base, 0.0004% w/w biotin, 1% v/v glycerol) with a single colony. Cultures were incubated with continuous shaking at 28°C for 24h. Cells were harvested by centrifugation at 2000xg for 10min and resuspended to an OD₆₀₀ of 1 in 500ml BMMY medium (1% w/w yeast extract, 2% w/w peptone, 100mM potassium phosphate pH 6, 1.34% w/w yeast nitrogen base, 0.0004% w/w biotin, 0.5% v/v MeOH) to induce expression. *P. pastoris* cells were incubated for 72h at 28°C with continuous shaking. Every 24h 100% methanol was added to a final concentration of 0.5% to maintain induction. Cells were harvested by centrifugation for 10min and 3000xg. The supernatants were transferred to a new tube and subsequently used for protein purification.

Solid ammonium sulfate was added to the supernatant of the *Pichia pastoris* cell culture to a final concentration of 40% w/w and stirred for 30min at room temperature (RT). After centrifugation at 10000xg for 15min at 4°C the pellet was dissolved in 5ml binding buffer (0.02M NaH₂PO₄, 1 M NaCl, 0.04M imidazol, 5% v/v glycerol, pH 7.5). The protein solution was filtered through a syringe-attached 0.45µm filter and loaded onto a 5ml Protino® Ni-NTA column (Macherey-Nagel, Düren, Germany) attached to an Akta Prime™ Plus (GE Healthcare, Little Chalfont, UK) chromatographic system. The column was washed with 150ml binding buffer to remove unbound proteins. The His-tagged fusion protein was eluted with 10ml 0.02M NaH₂PO₄, 1 M NaCl, 0.25M imidazol, 5% v/v glycerol, pH 7.5. Fractions containing the fusion-protein were combined and the elution buffer was replaced with 0.01M Tris/HCl pH 7.5, 5% v/v glycerol using PD-10 desalting columns (GE Healthcare, Little Chalfont, UK) according to the manufacturer's guidelines. Eluted fusion-protein was concentrated 10-fold by ultrafiltration using Amicon® Ultra centrifugal filters (Millipore, Billerica, MS, USA). Protein concentration was estimated using Qubit® Protein Assay (Invitrogen™, Carlsbad, USA) and BSA (bovine serum albumin) as standard. Proteins were snap-frozen in liquid nitrogen and stored at -80°C. Purification of fusion proteins were checked on SDS-PAGE using Any kd™ mini-protean® TGX™ precast polyacrylamide gels (Bio-Rad, Hercules, California, USA) according to the manufactures guidelines. Western blot analysis was performed using 10ng of purified protein and anti-c-myc antibody (Sigma Aldrich, St. Louis, MO, USA) following the protocol supplied with the antibody.

### Example 4: Inhibition assays

All inhibition assays were performed with purified recombinant inhibitor protein of example 3 on commercially available enzymes using colorimetric assays. Enzymes were pre-incubated without inhibitor protein (control) and with single and double amounts of inhibitor protein as specified below. After pre-incubation the enzymatic reactions were started by adding the substrate. Absorbance was recorded in a microplate reader (Synergy 4, BioTek, Winooski, VT, USA) and used to calculate the residual enzyme activity. Enzyme inhibition was expressed as percentage of the enzyme activity in the controls (100%). In a first round of experiments, three to five inhibitors were pooled in different molar ratios of inhibitor and enzyme and tested for enzyme inhibition. In case inhibitory activity of a pool on the tested enzyme was detected, the inhibitors composing the pool were re-tested individually and values for the half maximal inhibitory concentration (IC₅₀) were calculated. IC₅₀ values of active inhibitors were determined in triplicate by plotting percent initial activity against inhibitor concentrations from 0µM to 8µM in independent experiments. Results are shown in Table 1.

### Trypsin

Trypsin (EC 3.4.21.4) was purchased from Sigma Aldrich (St. Louis, USA). The trypsin inhibition assay was performed according to Heibges et al., 2003 with minor modifications using azocasein as substrate. Trypsin stock solution (5mg/ml) was prepared in a buffer containing 50mM Tris /HCl pH 7.5 and 20mM CaCl₂. 5µg trypsin was pre-incubated for 10min at RT either without (control), or with 0.5µM and 1µM purified inhibitor in 40µl 100mM Tris/HCl pH 7.5, 40mM CaCl₂. After adding 40µl 2% azocasein, the reactions were mixed by pipetting and incubated for 1h at RT. Reactions were stopped by adding 80µl 12% TCA and incubation for 30min at RT. Precipitate was removed by centrifugation and 100µl supernatant were transferred to a 96-well plate. 50µl 4N NaOH were added and the absorbance at 440nm was measured.

### β-Secretase (BACE-1)

Screening for BACE (EC 3.4.23.46) inhibitors were performed using the BACE Inhibitor Screening assay kit (Cayman Chemical Company, Ann Arbor, MI, USA) according to the manufacturer's instructions. BACE was pre-incubated for 15min at 4°C with either none (control), 100nM or 200nM inhibitor protein.

### Human HIV1-Protease

Screening for HIV-1 protease (EC 3.4.23.16) inhibitors was performed using the Sensolyte® 490 HIV1 Protease Assay Kit (Ana Spec, Fremont, CA, USA) according to the manufacturer's guidelines. HIV-1 protease was purchased from Ana Spec (Fremont, CA, USA) and was diluted to 10ng/µl with assay buffer complemented with dithiothreitol (DTT) supplied with the kit. HIV-1 protease was pre-incubated for 15min at room temperature with either none (control), 100nM or 200nM inhibitor protein.

### Cathepsin K

Screening for cathepsin K (EC 3.4.22.38) inhibitors was performed using the Cathepsin K Drug Discovery Kit (Enzo® Life Sciences, Farmingdale, New York, USA) according to the manufacturer's guidelines. Cathepsin K was pre-incubated for 30min at 37°C with either none(control), 100nM or 200nM inhibitor protein.

### 5-Lilpoxygenase (5-Lox)

Lipoxygenase (EC 1.13.11.34; 5-Lox) activity was assayed using the Lipoxygenase Inhibitor Screening Assay Kit (Cayman Chemical Company, Ann Arbor, MI, USA) according to the manufactures guidelines. 5-Lipoxygenase from *Solanum tuberosum* (potato) was purchased from Cayman Chemical Company (Ann Arbor, MI, USA). Linolenic acid was used as substrate. Residual 5-Lox activity was determined after pre-incubation for 15min at 4°C with either none (control), 200nM or 400nM protease inhibitor. Data analysis was performed according to the instructions supplied with the assay kit.

**Table 1: Functional characterization of heterologous expressed potato tuber protease inhibitors**

| | **Inhibitor (given No. represents SEQ IDs)** | **Trypsin ¹** | **pig pancreas-Elastase** | **BACE (β-Secretase)** | **Cathepsin K** | **5-Lipoxy-genase** | **HIV-1 Protease** |
|---|---|---|---|---|---|---|---|
| KPI-B | 07 | 53 | - | + (IC50 0,81µM) | - | - | - |
| | 08 | 59 | - | (IC50 0,4µM) | - | - | - |
| | 09 | 65 | - | + (IC50 0,36µM) | - | - | - |
| | 10 | 55 | - | - | - | - | - |
| | 11 | 38 | - | + (IC50 0,47 µM) | - | - | - |
| | 12 | 61 | - | - | - | - | - |
| | 13 | 83 | - | - | - | - | - |
| KPI-C | 01 | 0 | - | - | + (IC50 0,09µM) | + (IC50 1,59µM) | - |
| | 02 | 5 | - | + (IC50 0,72µM) | + (IC50 0,08µM) | + (IC50 1,01µM) | - |
| | 03 | 5 | - | - | + (IC50 0,02µM) | + (IC50 1,28µM) | - |
| PIN I | 04 | 44 | - | - | + (IC50 0,77 µM) | - | + (IC 50 0,67µM) |
| PIN II | 05 | 43 | + (IC50 0,67µM) | - | - | - | - |
| | 06 | 87 | + (IC50 0,97µM) | - | - | - | - |

| | | | | | | | |
|---|---|---|---|---|---|---|---|
| ¹ % Inhibition, molar ratio 2:1 Inhibitor/Trypsin | | | | | | | |

It has been shown previously, that many inhibitors from the KPI, PIN and PMEI families inhibit trypsin (Bauw et al., 2006; Heibges et al., 2003; Jorgensen et al., 2006; Pouvreau et al., 2001). Accordingly, the trypsin assay was used as a positive control for functional heterologous expression. The serine proteases dipeptidylpeptidase 4, factor IXa, factor Xa and thrombin were not affected by any of the tested inhibitors (not shown).

The KPI-B inhibitors (inhibitor 07, inhibitor 08, inhibitor 09, and inhibitor 11) and one KPI-C inhibitor (inhibitor 02) strongly inhibited BACE with IC₅₀ values between 0.36µM and 0.81 µM. The cysteine protease cathepsin K was effectively inhibited by all three KPI-C proteins tested (inhibitor 01, inhibitor 02 and inhibitor 03) and the PIN I inhibitor (inhibitor 04) which in addition inhibited the HIV-1 protease. This is very surprisingly because these inhibitors show no homology to known cysteine protease inhibitor super families. The KPI-C inhibitors with IC₅₀ values between 0.02µM and 0.09µM were more effective than the PIN I inhibitor with an IC₅₀ value of 0.77µM.

5-Lipoxygenase (EC 1.13.11.34) was the only non proteolytic enzyme which was inhibited by all three KPI-C inhibitors (inhibitor 01, inhibitor 02 and inhibitor 03) with IC₅₀ values of 1.59µM, 1.01 µM and 1.28µM, respectively. This shows that even inhibitory activity beyond protease inhibition is possible.

Two representatives from the potato proteinase inhibitor family II (PIN II) inhibited trypsin and in addition elastase from pig pancreas (PPE). The activity of human leukocyte elastase was not affected.

## Claims

1. Potato tuber derived proteinaceous protease inhibitor for use as an inhibitor of cathepsin K, 5-lipoxygenase, HIV-protease and/or human β-secretase.

2. The protease inhibitor according to claim 1 for use in the treatment of osteoporosis, allergies, asthma bronchiale, HIV-infection, and Alzheimer's disease.

3. The protease inhibitor according to claim 1 or 2, wherein the protease inhibitor is a KPI-C inhibitor.

4. The protease inhibitor according to claim 3 for the use as an inhibitor of cathepsin K or 5-lipoxygenase.

5. The protease inhibitor according to claim 3 or 4 for the use in the treatment of osteoporosis, allergies or asthma bronchiale.

6. The protease inhibitor according to claim 1 or 2, wherein the protease inhibitor is a PIN I inhibitor.

7. The protease inhibitor according to claim 6 for the use as an inhibitor of HIV-protease or cathepsin K.

8. The protease inhibitor according to claim 6 or 7 for the use in the treatment of HIV-infection or osteoporosis.

9. The protease inhibitor according to claim 1 or 2, wherein the protease inhibitor is a KPI-B inhibitor.

10. The protease inhibitor according to claim 9 for the use as an inhibitor of human β-secretase.

11. The protease inhibitor according to claim 9 or 10 for the use in the treatment Alzheimer's disease.

12. Use of a PIN II inhibitor or a KPI-C inhibitor as pesticide.

13. Cosmetic formulation containing at least one PIN I inhibitor, PIN II inhibitor, KPI-B inhibitor or one KPI-C inhibitor.

14. Potato tuber derived proteinaceous protease inhibitor with amino acid sequence of Seq ID No. 2, Seq ID No. 4, Seq ID No. 7, Seq ID No. 11, Seq ID No. 12 or Seq ID No. 13.

15. A method for designing a compound that interacts with or inhibits cathepsin K, 5-lipoxygenase, HIV-protease, and/or human β-secretase, comprising the steps of
(a) generating a three-dimensional model of potato tuber derived proteinaceous protease inhibitor according to anyone of SEQ ID No. 1 - SEQ ID No. 14; and
(b) employing said three-dimensional model to design a compound that should have the ability to interact with or inhibit cathepsin K, 5-lipoxygenase, HIV-protease, and/or human β-secretase.


SEQUENCE LISTING

<110>  MAX-PLANCK-GESELLSCHAFT zur FÖRDERUNG der WISSENSCHAFTEN
       e.V.

<120>  Novel uses of potato tuber proteinaceous inhibitors

<130>  GAR-P03669EP

<160>  41

<170>  PatentIn version 3.5

<210>  1
<211>  221
<212>  PRT
<213>  Solanum tuberosum


<220>
<221>  misc_feature
<222>  (211)..(211)
<223>  Xaa can be any naturally occurring amino acid

<400>  1

Met Lys Ser Ile Asn Ile Leu Ser Phe Leu Leu Leu Ser Ser Thr Leu
1               5                   10                  15


Ser Leu Val Ala Phe Ala Arg Ser Phe Thr Ser Glu Asn Pro Ile Val
            20                  25                  30


Leu Pro Thr Thr Cys His Asp Asp Asp Asn Leu Val Leu Pro Glu Val
        35                  40                  45


Tyr Asp Gln Asp Gly Asn Pro Leu Arg Ile Gly Glu Arg Tyr Ile Ile
    50                  55                  60


Lys Asn Pro Leu Leu Gly Ala Gly Ala Val Tyr Leu Asp Asn Ile Gly
65                  70                  75                  80


Asn Leu Gln Cys Pro Asn Ala Val Leu Gln His Met Ser Ile Pro Gln
                85                  90                  95


Phe Leu Gly Lys Gly Thr Pro Val Val Phe Ile Arg Lys Ser Glu Ser
            100                 105                 110


Asp Tyr Gly Asp Val Val Arg Leu Met Thr Ala Val Tyr Ile Lys Phe
        115                 120                 125


Phe Val Lys Thr Thr Lys Leu Cys Val Asp Glu Thr Val Trp Lys Val
    130                 135                 140


Asn Asn Glu Gln Leu Val Val Thr Gly Gly Asn Val Gly Asn Glu Asn

145                 150                 155                 160

Asp Ile Phe Lys Ile Lys Lys Thr Asp Leu Val Ile Arg Gly Met Lys
            165                 170                 175

Asn Val Tyr Lys Leu Leu His Cys Pro Ser His Leu Glu Cys Lys Asn
        180                 185                 190

Ile Gly Ser Asn Phe Lys Asn Gly Tyr Pro Arg Leu Val Thr Val Asn
    195                 200                 205

Asp Glu Xaa Asp Phe Ile Pro Phe Val Phe Ile Lys Ala
    210                 215                 220

<210>  2
<211>  246
<212>  PRT
<213>  Solanum tuberosum

<400>  2

Leu Ile Val Thr Cys Ser Leu Gln Gln Ile Asp Glu Gln Cys Phe Phe
1               5                   10                  15

Ile Met Pro Thr Leu Tyr Lys Lys Val Gly Ser Ile Asn Ile Leu Ser
            20                  25                  30

Phe Leu Leu Leu Ser Ser Thr Leu Ser Leu Val Ala Phe Ala Arg Ser
        35                  40                  45

Phe Thr Ser Glu Asn Pro Ile Val Leu Pro Thr Thr Cys His Asp Asp
    50                  55                  60

Asp Asn Leu Val Leu Pro Glu Val Tyr Asp Gln Asp Gly Asn Pro Leu
65                  70                  75                  80

Arg Ile Gly Glu Arg Tyr Ile Ile Lys Asn Pro Leu Leu Gly Ala Gly
                85                  90                  95

Ala Val Tyr Leu Asp Asn Ile Gly Asn Leu Gln Cys Pro Asn Ala Val
            100                 105                 110

Leu Gln His Met Ser Ile Pro Gln Phe Leu Gly Lys Gly Thr Pro Val
        115                 120                 125

Met Phe Val Arg Lys Ser Glu Ser Glu Ser Asp Tyr Gly Asp Val Val
    130                 135                 140

Arg Leu Met Thr Ala Val Tyr Ile Lys Phe Phe Val Lys Thr Thr Arg

145                 150                 155                 160

Phe Cys Val Lys Glu Thr Val Trp Lys Val Asn Asp Glu Gln Leu Val
                165                 170                 175

Val Thr Gly Gly Asn Val Gly Asn Glu Asn Asp Ile Phe Lys Ile Met
            180                 185                 190

Lys Thr Asp Leu Val Ile Arg Gly Met Lys Asn Val Tyr Lys Leu Leu
        195                 200                 205

His Cys Pro Ser His Leu Gln Cys Lys Asn Ile Gly Ser Asn Phe Lys
    210                 215                 220

Asn Gly Tyr Pro Arg Leu Val Thr Val Asp Gly Asp Asp Phe Ile Pro
225                 230                 235                 240

Phe Val Phe Ile Lys Ala
                245

<210>  3
<211>  217
<212>  PRT
<213>  Solanum tuberosum

<220>
<221>  misc_feature
<222>  (207)..(207)
<223>  Xaa can be any naturally occurring amino acid

<400>  3

Ser Ile Asn Ile Leu Ser Phe Leu Leu Leu Ser Ser Thr Leu Ser Leu
1               5                   10                  15

Val Ala Phe Ala Arg Ser Phe Thr Ser Glu Asn Pro Ile Val Leu Pro
            20                  25                  30

Thr Thr Cys His Asp Asn Leu Val Leu Pro Glu Val Tyr Asp Gln Asp
        35                  40                  45

Gly Asn Pro Leu Arg Ile Gly Glu Arg Tyr Ile Ile Lys Asn Pro Leu
    50                  55                  60

Leu Gly Ala Gly Ala Val Tyr Leu Asp Asn Ile Gly Asn Leu Gln Cys
65                  70                  75                  80

Pro Asn Ala Val Leu Gln His Met Ser Ile Pro Gln Phe Leu Gly Lys
                85                  90                  95

Gly Thr Pro Val Met Phe Val Arg Lys Ser Glu Ser Asp Asp Gly Asp
            100                 105                 110

Val Val Arg Leu Met Thr Ala Val Tyr Ile Lys Phe Phe Val Lys Thr
        115                 120                 125

Thr Lys Leu Cys Val Asp Glu Thr Val Trp Lys Val Asn Asp Glu Gln
    130                 135                 140

Leu Val Val Thr Gly Gly Asn Val Gly Asn Glu Asn Asp Ile Phe Lys
145                 150                 155                 160

Ile Lys Lys Thr Asp Leu Val Ile Arg Gly Met Lys Asn Val Tyr Lys
                165                 170                 175

Leu Leu His Cys Pro Ser His Leu Gln Cys Lys Asn Ile Gly Ser Asn
            180                 185                 190

Phe Lys Asn Gly Tyr Pro Arg Leu Val Thr Val Asp Asp Asp Xaa Asp
        195                 200                 205

Phe Ile Pro Phe Val Phe Ile Lys Ala
    210                 215

<210> 4
<211> 118
<212> PRT
<213> Solanum tuberosum

<400> 4

Lys Met Glu Gly Lys Ser Met Leu Lys Leu Ser His Val Leu Ala Phe
1               5                   10                  15

Leu Leu Leu Ala Ser Leu Phe Gln Ser Leu Met Ala Arg Asp Leu Ile
            20                  25                  30

Ser Asp Gly Ile Glu Val Leu Gln Leu Pro Val Glu Asn Glu Gly Glu
        35                  40                  45

Ser Val Phe Cys Pro Gly Lys Leu Ser Trp Pro Glu Leu Val Gly Met
    50                  55                  60

Pro Ala Gly Tyr Ala Lys Ala Ile Ile Glu Lys Glu Asn Ser Ile Val
65                  70                  75                  80

His Asp Val Arg Leu Leu Leu Pro Gly Met Val Lys Pro Ala Asn Tyr
                85                  90                  95

Val Cys Gly Arg Val Phe Leu Val Val Asp Leu Lys Leu Ile Val Gln
            100                 105                 110

Ile Pro Pro Ser Met Gly
        115

<210> 5
<211> 157
<212> PRT
<213> Solanum tuberosum

<400> 5

Leu Ser Ile Met Ala Val His Lys Glu Val Asn Phe Val Ala Tyr Leu
1               5                   10                  15

Leu Ile Val Leu Gly Leu Leu Val Leu Val Ser Ala Met Glu His Val
            20                  25                  30

Asp Ala Lys Ala Cys Thr Leu Glu Cys Gly Asn Leu Gly Phe Gly Ile
        35                  40                  45

Cys Pro Arg Ser Glu Gly Ser Pro Glu Asn Arg Ile Cys Thr Asn Cys
    50                  55                  60

Cys Ala Gly Tyr Lys Gly Cys Asn Tyr Tyr Ser Ala Asn Gly Ala Phe
65                  70                  75                  80

Ile Cys Glu Gly Glu Ser Asp Pro Lys Asn Pro Asn Val Cys Pro Arg
                85                  90                  95

Asn Cys Asp Thr Asn Ile Ala Tyr Ser Lys Cys Leu Arg Ser Glu Gly
            100                 105                 110

Lys Ser Leu Ile Tyr Pro Thr Glu Cys Thr Thr Cys Cys Thr Gly Tyr
        115                 120                 125

Lys Gly Cys Tyr Tyr Phe Gly Lys Asn Gly Lys Phe Val Cys Glu Gly
    130                 135                 140

Glu Ser Asp Glu Pro Lys Ala Asn Met Tyr Pro Ala Met
145                 150                 155

<210> 6
<211> 157
<212> PRT
<213> Solanum tuberosum

<400> 6

Leu Ser Ile Met Ala Val His Lys Glu Val Asn Phe Val Ala Tyr Leu
1               5                   10                  15

Leu Ile Val Leu Gly Leu Leu Val Leu Val Ser Ala Met Glu His Val
            20                  25                  30

Asp Ala Lys Ala Cys Thr Leu Glu Cys Gly Asn Leu Gly Phe Gly Ile
        35                  40                  45

Cys Pro Arg Ser Glu Gly Ser Pro Glu Asn Arg Ile Cys Thr Asn Cys
    50                  55                  60

Cys Ala Gly Tyr Lys Gly Cys Asn Tyr Tyr Ser Ala Asn Gly Ala Phe
65                  70                  75                  80

Ile Cys Glu Gly Glu Ser Asp Pro Lys Asn Pro Asn Val Cys Pro Arg
                85                  90                  95

Asn Cys Asp Thr Asn Ile Ala Tyr Ser Lys Cys Leu Arg Ser Glu Gly
            100                 105                 110

Lys Ser Leu Ile Tyr Pro Thr Glu Cys Thr Thr Cys Cys Thr Gly Tyr
        115                 120                 125

Lys Gly Cys Tyr Tyr Phe Gly Lys Asn Gly Lys Phe Val Cys Glu Gly
    130                 135                 140

Glu Ser Asp Glu Pro Lys Ala Asn Met Tyr Pro Ala Met
145                 150                 155

<210>  7
<211>  254
<212>  PRT
<213>  Solanum tuberosum

<400>  7

Leu Ile Val Thr Cys Ser Leu Gln Gln Ile Asp Glu Gln Cys Phe Phe
1               5                   10                  15

Ile Met Pro Thr Leu Tyr Lys Lys Val Gly Thr Asn Lys Ile Ser Ser
            20                  25                  30

Leu Asn Lys Met Lys Cys Leu Phe Leu Leu Cys Leu Cys Leu Val Pro
        35                  40                  45

Ile Val Val Phe Ser Ser Thr Phe Thr Ser Lys Asn Pro Ile Asn Leu
    50                  55                  60

Pro Ser Asp Ala Thr Pro Val Leu Asp Val Ala Gly Lys Glu Leu Asp
65                  70                  75                  80

Ser Arg Leu Ser Tyr Arg Ile Ile Ser Thr Phe Trp Gly Ala Leu Gly
                85                  90                  95

Gly Asp Val Tyr Leu Gly Lys Ser Pro Asn Ser Asp Ala Pro Cys Ala
            100                 105                 110

Asn Gly Ile Phe Arg Tyr Asn Ser Asp Val Gly Pro Ser Gly Thr Pro
        115                 120                 125

Val Arg Phe Ile Gly Ser Ser Ser His Phe Gly Gln Gly Ile Phe Glu
    130                 135                 140

Asn Glu Leu Leu Asn Ile Gln Phe Ala Ile Ser Thr Ser Lys Leu Cys
145                 150                 155                 160

Val Ser Tyr Thr Ile Trp Lys Val Gly Asp Tyr Asp Ala Ser Leu Gly
                165                 170                 175

Thr Met Leu Leu Glu Thr Gly Gly Thr Ile Gly Gln Ala Asp Ser Ser
            180                 185                 190

Trp Phe Lys Ile Val Lys Ser Ser Arg Phe Gly Tyr Asn Leu Leu Tyr
        195                 200                 205

Cys Pro Val Thr Ile Ile Cys Pro Ser Cys Pro Asp Asp Gln Phe Cys
    210                 215                 220

Ser Lys Val Gly Val Val His Gln Asn Gly Lys Arg Arg Leu Ala Leu
225                 230                 235                 240

Val Lys Asp Asn Pro Leu Asp Val Ser Phe Lys Gln Val Gln
                245                 250

<210>  8
<211>  256
<212>  PRT
<213>  Solanum tuberosum

<400>  8

Leu Ile Val Thr Cys Ser Leu Gln Gln Ile Asp Glu Gln Cys Phe Phe
1               5                   10                  15

Ile Met Pro Thr Leu Tyr Lys Lys Val Gly Thr Asn Lys Ile Ser Ser
            20                  25                  30

Leu Asn Lys Met Lys Cys Leu Phe Leu Leu Cys Leu Cys Leu Val Pro
        35                  40                  45

Ile Val Val Phe Ser Ser Thr Phe Thr Ser Gln Asn Pro Ile Asn Leu
    50                  55                  60

Pro Ser Asp Ala Thr Pro Val Leu Asp Val Thr Gly Lys Glu Leu Asp
65                  70                  75                  80

Pro Arg Leu Ser Tyr Arg Ile Ile Ser Thr Phe Trp Gly Ala Leu Gly
                85                  90                  95

Gly Asp Val Tyr Leu Gly Lys Ser Pro Asn Ser Asp Ala Pro Cys Ala
            100                 105                 110

Asn Gly Val Phe Arg Tyr Asn Ser Asp Val Gly Pro Ser Gly Thr Pro
        115                 120                 125

Val Arg Phe Ile Gly Ser Ser Ser His Phe Gly Gln Gly Ile Phe Glu
    130                 135                 140

Asp Glu Leu Leu Asn Ile Gln Phe Ala Ile Ser Thr Ser Lys Met Cys
145                 150                 155                 160

Val Ser Tyr Thr Ile Cys Lys Val Gly Asp Tyr Asp Ala Ser Leu Gly
                165                 170                 175

Thr Met Leu Leu Glu Thr Gly Gly Thr Ile Ser Gln Ala Asp Ser Ser
            180                 185                 190

Trp Phe Lys Ile Val Lys Ser Ser Gln Phe Gly Tyr Asn Leu Leu Tyr
        195                 200                 205

Cys Pro Val Thr Thr Thr Met Thr Leu Pro Phe Ser Ser Asp Asp Gln
    210                 215                 220

Phe Cys Leu Lys Val Gly Val Val His Gln Asn Gly Lys Arg Arg Leu
225                 230                 235                 240

Ala Leu Val Lys Asp Asn Pro Leu Asp Val Ser Phe Lys Gln Val Gln
                245                 250                 255

<210>  9
<211>  230
<212>  PRT
<213>  Solanum tuberosum

<400>  9

Thr Asn Lys Ile Ser Ser Leu Asn Lys Met Lys Cys Leu Phe Leu Leu
1               5                   10                  15

Cys Leu Cys Leu Val Pro Ile Val Val Phe Ser Ser Thr Phe Thr Ser
            20                  25                  30

Gln Asn Pro Ile Asn Leu Pro Ser Asp Ala Thr Pro Val Leu Asp Val
        35                  40                  45

Thr Gly Lys Glu Leu Asp Pro Arg Leu Ser Tyr Arg Ile Ile Ser Thr
    50                  55                  60

Phe Trp Gly Ala Leu Gly Gly Asp Val Tyr Leu Gly Lys Ser Pro Asn
65                  70                  75                  80

Ser Asp Ala Pro Cys Ala Asn Gly Val Phe Arg Tyr Asn Ser Asp Val
                85                  90                  95

Gly Pro Ser Gly Thr Pro Val Arg Phe Ile Gly Ser Ser Ser His Phe
            100                 105                 110

Gly Gln Gly Ile Phe Glu Asp Glu Leu Leu Asn Ile Gln Phe Ala Ile
        115                 120                 125

Ser Thr Ser Lys Met Cys Val Ser Tyr Thr Ile Trp Lys Val Gly Asp
    130                 135                 140

Tyr Asp Ala Ser Leu Gly Thr Met Leu Leu Glu Thr Gly Gly Thr Ile
145                 150                 155                 160

Gly Gln Ala Asp Ser Ser Trp Phe Lys Ile Val Lys Ser Ser Gln Phe
                165                 170                 175

Gly Tyr Asn Leu Leu Tyr Cys Pro Val Thr Thr Thr Met Thr Leu Pro
            180                 185                 190

Phe Ser Ser Asp Asp Gln Phe Cys Leu Lys Val Gly Val Val His Gln
        195                 200                 205

Asn Gly Lys Arg Arg Leu Ala Leu Val Lys Asp Asn Pro Leu Asp Val
    210                 215                 220

Ser Phe Lys Gln Val Gln
225                 230

<210>  10
<211>  228
<212>  PRT

<213>  Solanum tuberosum

<400>  10

Lys Ile Ser Ser Leu Asn Lys Met Lys Cys Leu Phe Leu Leu Cys Leu
1               5                   10                  15

Cys Leu Phe Pro Ile Val Val Phe Ser Ser Thr Phe Thr Ser Gln Asn
            20                  25                  30

Pro Ile Asn Leu Pro Ser Asp Ala Thr Pro Val Leu Asp Val Thr Gly
        35                  40                  45

Lys Glu Leu Asp Pro Arg Leu Ser Tyr Arg Ile Ile Ser Thr Phe Trp
    50                  55                  60

Gly Ala Leu Gly Gly Asp Val Tyr Leu Gly Lys Ser Pro Asn Ser Asp
65                  70                  75                  80

Ala Pro Cys Ala Asn Gly Val Phe Arg Tyr Asn Ser Asp Val Gly Pro
                85                  90                  95

Ser Gly Thr Pro Val Arg Phe Ile Gly Ser Ser Ser His Phe Gly Gln
            100                 105                 110

Gly Ile Phe Glu Asp Glu Leu Leu Asn Ile Gln Phe Ala Ile Ser Thr
        115                 120                 125

Ser Lys Met Cys Val Ser Tyr Thr Ile Trp Lys Val Gly Asp Tyr Asp
    130                 135                 140

Ala Ser Leu Gly Thr Met Leu Leu Glu Thr Gly Gly Thr Ile Gly Gln
145                 150                 155                 160

Ala Asp Ser Ser Trp Phe Lys Ile Val Lys Ser Ser Gln Phe Gly Tyr
                165                 170                 175

Asn Leu Leu Tyr Cys Pro Val Thr Thr Thr Met Thr Leu Pro Phe Ser
            180                 185                 190

Ser Asp Asp Gln Phe Cys Leu Lys Val Gly Val Val His Gln Asn Gly
        195                 200                 205

Lys Arg Arg Leu Ala Leu Val Lys Asp Asn Pro Leu Asp Val Ser Phe
    210                 215                 220

Lys Gln Val Gln
225

<210>  11
<211>  228
<212>  PRT
<213>  Solanum tuberosum

<400>  11

Thr Asn Lys Ile Ser Ser Phe Asn Lys Met Lys Cys Leu Phe Leu Leu
1               5                   10                  15

Cys Leu Cys Leu Val Pro Ile Val Val Phe Ser Ser Thr Phe Thr Ser
            20                  25                  30

Glu Asn Pro Ile Asn Leu Pro Ser Asp Ala Thr Pro Val Leu Asp Val
        35                  40                  45

Thr Gly Lys Glu Leu Asp Pro Arg Leu Ser Tyr Arg Ile Ile Ser Thr
    50                  55                  60

Phe Trp Gly Ala Leu Gly Gly Asp Val Tyr Leu Gly Lys Ser Pro Asn
65                  70                  75                  80

Pro Asp Ala Pro Cys Ala Asn Gly Val Phe Arg Tyr Asn Ser Asp Val
                85                  90                  95

Gly Pro Ser Gly Thr Pro Val Ile Phe Ile Gly Ser Ser Ser His Phe
            100                 105                 110

Gly Pro His Ile Phe Glu Asp Glu Leu Leu Asn Ile Gln Phe Ala Ile
        115                 120                 125

Ser Thr Ser Lys Leu Cys Val Ser Tyr Thr Ile Trp Lys Val Gly Asp
    130                 135                 140

Tyr Asp Ala Ser Leu Gly Thr Met Leu Leu Glu Thr Gly Gly Thr Ile
145                 150                 155                 160

Gly Gln Ala Asp Ser Ser Trp Phe Lys Ile Val Lys Ser Ser Arg Phe
                165                 170                 175

Gly Tyr Asn Leu Leu Tyr Cys Pro Val Thr Ile Ile Cys Pro Ser Cys
            180                 185                 190

Pro Asp Asp Gln Phe Cys Ser Lys Val Gly Val Val His Gln Asn Gly
        195                 200                 205

Lys Arg Arg Leu Ala Leu Val Lys Asp Asn Pro Leu Asp Val Ser Phe
    210                 215                 220

Lys Gln Val Gln
225

<210> 12
<211> 213
<212> PRT
<213> Solanum tuberosum

<400> 12

Met Lys Cys Leu Phe Leu Leu Cys Leu Cys Leu Val Pro Ile Val Val
1               5                   10                  15

Phe Ser Ser Thr Phe Thr Ser Glu Asn Pro Ile Asn Leu Pro Ser Asp
            20                  25                  30

Ala Thr Pro Val Leu Asp Val Thr Gly Lys Glu Leu Asp Pro Arg Leu
        35                  40                  45

Ser Tyr Arg Ile Ile Ser Thr Phe Trp Gly Ala Leu Gly Gly Asp Val
    50                  55                  60

Tyr Leu Gly Lys Ser Pro Asn Ser Asp Ala Pro Cys Ala Asn Gly Val
65                  70                  75                  80

Phe Arg Tyr Asn Ser Asp Val Gly Pro Ser Gly Thr Pro Val Arg Phe
                85                  90                  95

Ile Gly Ser Ser Ser His Phe Gly Pro His Ile Phe Glu Asp Glu Leu
            100                 105                 110

Leu Asn Ile Gln Phe Ala Ile Ser Thr Ser Lys Leu Cys Val Ser Tyr
        115                 120                 125

Thr Ile Trp Lys Val Gly Asp Tyr Asp Ala Ser Leu Gly Thr Met Leu
    130                 135                 140

Leu Glu Thr Gly Gly Thr Ile Gly Gln Ala Asp Ser Ser Trp Phe Lys
145                 150                 155                 160

Ile Val Lys Ser Ser Arg Phe Gly Tyr Asn Leu Leu Tyr Cys Pro Val
                165                 170                 175

Thr Ile Ile Cys Pro Ser Cys Pro Asp Asp Gln Phe Cys Ser Lys Val
            180                 185                 190

Gly Val Val His Gln Asn Gly Lys Arg Arg Leu Ala Leu Val Lys Asp
        195                 200                 205

Asn Pro Leu Asp Val
    210

<210>  13
<211>  213
<212>  PRT
<213>  Solanum tuberosum

<400>  13

Met Lys Cys Leu Phe Leu Leu Cys Leu Cys Leu Val Pro Ile Val Val
1               5                   10                  15

Phe Ser Ser Thr Phe Thr Ser Gln Asn Pro Ile Asn Leu Pro Ser Asp
            20                  25                  30

Ala Thr Pro Val Leu Asp Val Thr Gly Lys Glu Leu Asp Pro Arg Leu
        35                  40                  45

Ser Tyr Arg Ile Ile Ser Ile Gly Arg Gly Ala Leu Gly Gly Asp Val
    50                  55                  60

Tyr Leu Gly Lys Ser Pro Asn Ser Asp Ala Pro Cys Ala Asn Gly Val
65                  70                  75                  80

Phe Arg Tyr Asn Ser Asp Val Gly Pro Ser Gly Thr Pro Val Arg Phe
                85                  90                  95

Ile Gly Ser Ser Ser His Phe Gly Pro His Ile Phe Glu Asp Glu Leu
            100                 105                 110

Leu Asn Ile Gln Phe Ala Ile Ser Thr Ser Lys Leu Cys Val Ser Tyr
        115                 120                 125

Thr Ile Trp Lys Val Gly Asp Tyr Asp Ala Ser Leu Gly Thr Met Leu
    130                 135                 140

Leu Glu Thr Gly Gly Thr Ile Gly Gln Ala Asp Ser Ser Trp Phe Lys
145                 150                 155                 160

Ile Val Lys Ser Ser Gln Leu Gly Tyr Asn Leu Leu Tyr Cys Pro Phe
                165                 170                 175

Ser Ser Asp Asp Gln Phe Cys Leu Lys Val Gly Val Val His Gln Asn
            180                 185                 190

Gly Lys Arg Arg Leu Ala Leu Val Lys Asp Asn Pro Leu Asp Val Ser
        195                 200                 205

Phe Lys Gln Val Gln
    210

<210>  14
<211>  34
<212>  DNA
<213>  Artificial Sequence

<220>
<223>  forward primer for cloning inhibitor 02 in pPICZalphaA

<400>  14
aaacacgtgc gaaaagaaag tgatggacta gaag                                    34

<210>  15
<211>  33
<212>  DNA
<213>  Artificial Sequence

<220>
<223>  reverse primer for cloning inhibitor 02 in pPICZalphaA

<400>  15
aaagcggccg caccaaccac aggaatttgt aca                                     33

<210>  16
<211>  37
<212>  DNA
<213>  Artificial Sequence

<220>
<223>  forward primer for cloning inhibitor 03 in pPICZalphaA

<400>  16
aaagaattca tgcttgtact ccctgaagtt tatgacc                                 37

<210>  17
<211>  31
<212>  DNA
<213>  Artificial Sequence

<220>
<223>  reverse primer for cloning inhibitor 03 in pPICZalphaA

<400>  17
aaagcggccg ccgccttgat gaacacaaat g                                       31

<210>  18
<211>  32
<212>  DNA
<213>  Artificial Sequence

<220>
<223>  forward primer for cloning inhibitor 04 in pPICZalphaA

<400>  18
aaagaattca gagatttgat cagtgatggc at                                      32

<210>  19
<211>  32
<212>  DNA
<213>  Artificial Sequence

<220>
<223>  reverse primer for cloning inhibitor 04 in pPICZalphaA

<400>  19
aaagcggccg cacccatact gggaggaatt tg                                    32


<210>  20
<211>  32
<212>  DNA
<213>  Artificial Sequence

<220>
<223>  forward primer for cloning inhibitor 05 in pPICZalphaA

<400>  20
aaagaattca tgaaggcttg cactttagaa tg                                    32


<210>  21
<211>  33
<212>  DNA
<213>  Artificial Sequence

<220>
<223>  reverse primer for cloning inhibitor 05 in pPICZalphaA

<400>  21
aaagcggccg ccattgcagg gtacatattt gcc                                   33


<210>  22
<211>  32
<212>  DNA
<213>  Artificial Sequence

<220>
<223>  forward primer for cloning inhibitor 06 in pPICZalphaA

<400>  22
aaagaattca tgaaggcttg cactttagaa tg                                    32


<210>  23
<211>  33
<212>  DNA
<213>  Artificial Sequence

<220>
<223>  reverse primer for cloning inhibitor 06 in pPICZalphaA

<400>  23
aaagcggccg ccattgcagg gtacatattt gcc                                   33


<210>  24

<211>  17
<212>  DNA
<213>  Artificial Sequence

<220>
<223>  primer used for sequencing of insertions in pDONR\231222

<400>  24
gtaaaacgac ggccagt                                                    17


<210>  25
<211>  39
<212>  DNA
<213>  Artificial Sequence

<220>
<223>  forward primer for cloning inhibitor 07 in pPICZalphaA

<400>  25
aaagaattca tgctacctag tgatgctact ccagtactt                            39


<210>  26
<211>  32
<212>  DNA
<213>  Artificial Sequence

<220>
<223>  reverse primer for cloning inhibitor 07 in pPICZalphaA

<400>  26
aaagcggccg cctggacttg cttgaaggag ac                                   32


<210>  27
<211>  32
<212>  DNA
<213>  Artificial Sequence

<220>
<223>  forward primer for cloning inhibitor 08 in pPICZalphaA

<400>  27
aaagaattca tgccagtact tgacgtaact gg                                   32


<210>  28
<211>  32
<212>  DNA
<213>  Artificial Sequence

<220>
<223>  reverse primer for cloning inhibitor 08 in pPICZalphaA

<400>  28
aaagcggccg cctggacttg cttgaaggag ac                                   32


<210>  29
<211>  32
<212>  DNA
<213>  Artificial Sequence

<220>
<223>  forward primer for cloning inhibitor 09 in pPICZalphaA

<400>  29
aaagaattca tgccagtact tgacgtaact gg                                    32


<210>  30
<211>  32
<212>  DNA
<213>  Artificial Sequence

<220>
<223>  reverse primer for cloning inhibitor 09 in pPICZalphaA

<400>  30
aaagcggccg cctggacttg cttgaaggag ac                                    32


<210>  31
<211>  32
<212>  DNA
<213>  Artificial Sequence

<220>
<223>  forward primer for cloning inhibitor 10 in pPICZalphaA

<400>  31
aaagaattca tgccagtact tgacgtaact gg                                    32


<210>  32
<211>  32
<212>  DNA
<213>  Artificial Sequence

<220>
<223>  reverse primer for cloning inhibitor 10 in pPICZalphaA

<400>  32
aaagcggccg cctggacttg cttgaaggag ac                                    32


<210>  33
<211>  32
<212>  DNA
<213>  Artificial Sequence

<220>
<223>  forward primer for cloning inhibitor 11 in pPICZalphaA

<400>  33
aaagaattca tgccagtact tgacgtaact gg                                    32


<210>  34
<211>  32
<212>  DNA
<213>  Artificial Sequence

<220>
<223>  reverse primer for cloning inhibitor 11 in pPICZalphaA

<400>  34
aaagcggccg cctggacttg cttgaaggag ac                                    32

<210>  35
<211>  16
<212>  DNA
<213>  Artificial Sequence

<220>
<223>  primer used for sequencing of insertions in pDONR\231222 3' end

<400>  35
aacagctatg accatg                                                      16

<210>  36
<211>  34
<212>  DNA
<213>  Artificial Sequence

<220>
<223>  forward primer for cloning inhibitor 01 in oPICZalphaA

<400>  36
aaagaattca tgcttgtact ccctgaagtt tatg                                  34

<210>  37
<211>  31
<212>  DNA
<213>  Artificial Sequence

<220>
<223>  reverse primer for cloning inhibitor 01 in oPICZalphaA

<400>  37
aaagcggccg ccgccttgat gaacacaaat g                                     31

<210>  38
<211>  32
<212>  DNA
<213>  Artificial Sequence

<220>
<223>  forward primer for cloning inhibitor 12 in oPICZalphaA

<400>  38
aaagaattca tgccagtact tgacgtaact gg                                    32

<210>  39
<211>  32
<212>  DNA
<213>  Artificial Sequence

<220>
<223>  reverse primer for cloning inhibitor 12 in oPICZalphaA

<400>  39
aaagcggccg cctggacttg cttgaaggag ac                                    32

<210>  40
<211>  32
<212>  DNA
<213>  Artificial Sequence

<220>
<223>  forward primer for cloning inhibitor 13 in oPICZalphaA

<400>  40
aaagaattca tgccagtact tgacgtaact gg                                    32


<210>  41
<211>  32
<212>  DNA
<213>  Artificial Sequence

<220>
<223>  reverse primer for cloning inhibitor 13 in oPICZalphaA

<400>  41
aaagcggccg cctggacttg cttgaaggag ac                                    32